# EUROPÄISCHE PATENTSCHRIFT

(11) **EP 0 518 079 B1**
(45) Veröffentlichungstag und Bekanntmachung des Hinweises auf die Patenterteilung: **20.11.1996**
(21) Anmeldenummer: 92108309.3
(22) Anmeldetag: 16.05.1992
(51) Int. Cl.: A61B 17/58, F16B 43/02, F16B 33/00

(54) **Osteosyntheseimplantat**
Implant for osteosynthesis
Implant pour ostéosynthèse

(30) Priorität: 13.06.1991 DE 4119447
(43) Veröffentlichungstag der Anmeldung: 16.12.1992
(73) Patentinhaber: AESCULAP AG, D-78501 Tuttlingen (DE)
(72) Erfinder: Breiter, Werner, W-7755 Dauchingen (DE)
(74) Vertreter: Hoeger, Stellrecht & Partner

(56) Entgegenhaltungen:
- EP-A- 0 048 038
- WO-A-86/00118
- CH-A- 611 147
- DE-C- 251 246
- DE-U- 8 623 003
- Technica Reihe Nr.2, Heft 2 Allgemeine Gestaltungslehre Birkhäuser Verlag Basel und Stuttgart ALBERT LEYER: "Maschinenkonstruktionslehre"

## Beschreibung

Die Erfindung betrifft ein Osteosyntheseimplantat mit einer Knochenplatte mit Durchstecköffnungen und mit schrauben-, zuganker- oder stiftförmigen Spannelementen, die in die Durchstecköffnungen eingesetzt sind und mit einem verbreiterten Kopf am Rand der Durchstecköffnungen anliegen und dadurch die Knochenplatte gegen einen Knochen spannen.

Derartige Implantate werden seit langer Zeit erfolgreich zur Fixierung von Knochenfragmenten verwendet. Im Bereich der Durchstecköffnungen ist der Querschnitt der Knochenplatten geschwächt, es ergeben sich daher über die Länge der Knochenplatten ungleiche Festigkeitseigenschaften. Die Festigkeit im Bereich der Durchstecköffnungen wird insbesondere auch dadurch geschwächt, daß herkömmliche Durchstecköffnungen konisch ausgebildet sind (EP 048 038 A1). Die in diese konischen Durchstecköffnungen eingreifenden Köpfe der Spannelemente drücken die Randbereiche der Durchstecköffnungen auseinander, es erfolgt also eine Dehnung und Spreizung im Bereich der Durchstecköffnungen, die insbesondere bei Verwendung von Knochenplatten aus Kunststoff zu Schwächungen führt.

Es ist Aufgabe der Erfindung, ein Osteosyntheseimplantat zu schaffen, bei dem die Querschnittsschwächung im Bereich der Durchstecköffnungen nicht zu einer Abnahme der Festigkeit des eingesetzten Implantates führt, insbesondere nicht unter dem Einfluß der gespannten Spannelemente, und bei dem unter Beibehaltung dieser Eigenschaft auch bei schräg eingesetzten Spannelementen eine allseitige Anlage zwischen Anlagefläche und Spannfläche und damit die gewünschte Beaufschlagung des Randes der Durchstecköffnung mit nach innen gerichteten Kräften aufrechterhalten werden.

Diese Aufgabe wird bei einem Osteosyntheseimplantat der eingangs beschriebenen Art erfindungsgemäß dadurch gelöst, daß die Ränder der Durchstecköffnungen radial nach außen abfallende Anlageflächen bilden, daß die verbreiterten Köpfe der Spannelemente über eine radial nach außen abfallende Spannfläche an den Anlageflächen anliegen, daß der Außenumfang der Anlagefläche größer ist als der Außenumfang der Spannfläche und daß das Spannelement die Durchstecköffnung mit so viel Spiel durchsetzt, daß es gegenüber der Senkrechten auf der Knochenplatte mit einer Neigung, bei der sich trotzdem eine allseitige Anlage zwischen Anlagefläche und Spannfläche ergibt, in die Durchstecköffnung einsetzbar ist.

Durch diese Anordnung werden bei gespannten Spannelementen radial nach innen gerichtete Kräfte auf den Randbereich der Durchstecköffnungen ausgeübt, die im Gegensatz zu bekannten konischen Durchstecköffnungen das Material im Durchstecköffnungsbereich nicht schwächen, sondern dazu beitragen, daß die Festigkeit im Durchstecköffnungsbereich erhöht wird. Tatsächlich tragen auf diese Weise die Köpfe der Spannelemente zur Festigkeit im Durchstecköffnungsbereich bei, da die Köpfe nach außen gerichtete Kräfte aufnehmen. Dadurch wird ein Teil der Materialschwächung im Öffnungsbereich praktisch wieder ausgeglichen, die Köpfe übernehmen einen Teil der Festigkeitswirkung des im Durchstecköffnungsbereich fehlenden Materials.

Bei der beschriebenen Anordnung ergibt sich eine Zentrierwirkung beim Einsetzen der Spannelemente, die vorteilhaft ist und ermöglicht, die Schrauben mit relativ geringer Präzision zu setzen. Auch bei einem schrägen Einsetzen des Spannelements, bei dem dieses gegenüber der auf der Knochenplatte stehenden Senkrechten eine geringe Neigung aufweist, überdecken sich die Anlagefläche und die Spannfläche auf einer Seite des Spannelements mehr als auf der gegenüberliegenden Seite. Trotzdem ergibt sich eine allseitige Anlage zwischen Anlagefläche und Spannfläche und damit die gewünschte Beaufschlagung des Randes der Durchstecköffnung mit nach innen gerichteten Kräften.

Es ist zwar allgemein im Maschinenbau bekannt, an Köpfen von Schrauben radial nach außen abfallende Spannflächen und an von ihnen gespannten Teilen nach außen abfallende Anlageflächen vorzusehen, jedoch handelt es sich dabei um Maschinenbauteile und nicht um Osteosyntheseimplantate. Außerdem sind bei diesen bekannten Konstruktionen die Spannelemente genau parallel zur Achse der Durchstecköffnung ausgerichtet, eine Neigung ist daher nicht möglich (WO86/00118).

Bei dem vorliegenden Osteosyntheseimplantat können die Spannflächen Teil des Kopfes des Spannelements sein, es kann aber auch vorgesehen sein, daß die Spannflächen Teil eines Ringes sind, der zwischen Anlagefläche und Kopf des Spannelements eingelegt ist. Bei einem besonderen Ausführungsbeispiel kann der Ring mit der Unterseite des Kopfes des Spannelements dauerhaft verbunden sein, beispielsweise durch Verkleben oder Verschweißen.

Günstig ist es, wenn die Spannfläche stärker abfällt als die Anlagefläche. Dadurch ist sichergestellt, daß auch bei nicht genauer Zentrierung das Spannelement immer an der Anlagefläche anliegt und diese mit einer radial nach innen gerichteten Kraft beaufschlagt.

Es ist aber auch möglich, die Neigung der Anlagefläche und der Spannfläche gleich zu wählen.

Es ist möglich, daß die Anlagefläche gegenüber der Außenfläche der Knochenplatte in axialer Richtung des Spannelements zurückgesetzt ist. Dadurch steht der Kopf eines eingesetzten Spannelements nicht so weit über die Anlagefläche der Knochenplatte vor, dies ist bei der Implantation von Vorteil.

Bei einer bevorzugten Ausführungsform ist vorgesehen, daß die Knochenplatte auf ihrer Unterseite Bereiche geringerer Plattenstärke aufweist, die zwischen benachbarten Durchstecköffnungen angeordnet sind, sich jedoch nicht bis in den Bereich des Randes der Durchstecköffnungen und nicht bis in den Bereich des Längsrandes der Knochenplatte erstrecken. Dadurch liegt die Knochenplatte auf dem Knochen nicht flächig auf, sondern längs des die Durchstecköffnungen umgebenden Randes und längs des Längsrandes der Knochenplatte. Dies führt einerseits zu einer besseren Fixierung der Knochenplatte am Knochen, andererseits ermöglicht es eine wirksamere Ernährung der Knochenbereiche, an denen keine Knochenplatte anliegt, das heißt die Schädigungsgefahr des Knochens bei längerer Anlage von Knochenplatten wird herabgesetzt. Durch diese Ausgestaltung kann außerdem erreicht werden, daß die mechanische Festigkeit der Knochenplatte über ihre gesamte Länge vergleichsmäßig wird, da das fehlende Material im Bereich der Durchstecköffnungen teilweise durch die größere Plattenstärke in diesem Bereich ausgeglichen wird.

Günstig ist es, wenn die Knochenplatte und die Spannelemente aus Kunststoff bestehen, insbesondere aus resorbierbarem Kunststoff.

Der Ausdruck "Knochenplatten" erfaßt alle flächigen Implantate, die an Knochenfragmente anlegbar sind und Durchstecköffnungen für Spannelemente aufweisen. Der Ausdruck Spannelemente erfaßt Knochenschrauben, die in das Knochenmaterial einschraubbar sind, sowie alle Befestigungsmittel, mit denen Knochenplatten am Knochen festgelegt werden können. Dazu gehören beispielsweise auch Zuganker oder Fixierungsstifte, die in anderer Weise als durch Einschrauben in den Knochen gespannt werden. Wesentlich ist dabei lediglich, daß diese Spannelemente mit Hilfe eines Kopfes gegen die Außenseite der Knochenplatte gedrückt werden und die Knochenplatte dadurch gegen die Oberfläche des Knochens spannen.

Die nachfolgende Beschreibung bevorzugter Ausführungsformen dient im Zusammenhang mit der Zeichnung der näheren Erläuterung. Es zeigen:
- Figur 1 :: eine in Längsrichtung geschnittene Knochenplatte aus einem resorbierbaren Kunststoffmaterial;
- Figur 2 :: eine Ansicht der Knochenplatte der Figur 1 von der Unterseite her und
- Figur 3 :: eine Schnittansicht einer an einem Röhrenknochen angelegten Knochenplatte längs Linie 3-3 in Figur 2 mit gespannter Knochenschraube.

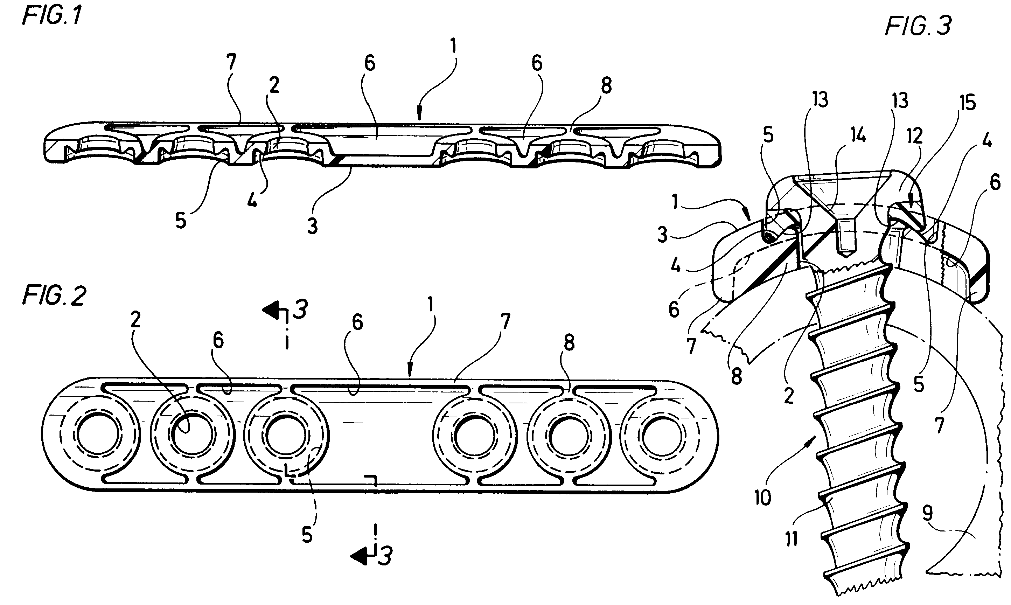

Die in der Zeichnung dargestellte Knochenplatte 1 ist länglich ausgebildet und weist an beiden Enden je drei in Längsrichtung nebeneinander angeordnete, kreisförmige Querschnitte aufweisende Durchstecköffnungen 2 auf. Auf der Außenseite 3 der Knochenplatte 1 (in Figur 1 unten) wird jede Durchstecköffnung 2 von einer radial nach außen schräg abfallenden Anlagefläche 4 umgeben, die am äußeren Rand in eine senkrechte, bis zur Außenseite 3 führende Wand 5 übergeht.

Auf der Unterseite der Knochenplatte 1 ist diese im Bereich zwischen den Durchstecköffnungen 2 mit zur Unterseite offenen Ausnehmungen 6 versehen, so daß die Knochenplatte 1 in diesen Bereichen eine geringere Materialstärke aufweist. Diese Ausnehmungen 6 erstrecken sich nicht in den Bereich der Längskanten 7 der Knochenplatte und nicht in den die Durchstecköffnungen 2 umgebenden Randbereich, so daß in diesen Bereichen die Knochenplatte 1 eine größere Materialstärke aufweist. Eine größere Materialstärke ist auch in quer zur Längsrichtung der Knochenplatte 1 verlaufenden Stegen 8 vorhanden, die in Höhe der Durchstecköffnungen 2 angeordnet sind und benachbarte Ausnehmungen 6 voneinander trennen.

Die Knochenplatte besteht vorzugsweise aus einem thermoplastischen Kunststoffmaterial, insbesondere aus einem resorbierbaren Kunststoff. Wie aus Figur 3 ersichtlich ist, ist sie zur besseren Anpassung an Knochenflächen quer zur Längsrichtung gebogen ausgebildet.

Zur Festlegung einer Knochenplatte 1 an einer Knochenoberfläche wird die Knochenplatte 1 auf die Oberfläche des Knochens 9 aufgelegt, wobei sie mit den Unterseiten der Längskanten 7, der Stege 8 und der die Durchstecköffnungen 2 umgebenden Ränder auf dem Knochen 9 aufliegt, während die Knochenplatte im Bereich der Ausnehmungen 6 im Abstand von der Oberfläche des Knochens 9 verläuft (Figur 3).

Zur Festlegung der Knochenplatte 1 am Knochen sind Knochenschrauben 10 durch jede Durchstecköffnung 2 hindurchgesteckt, die anschließend in den Knochen 9 eingeschraubt sind. Diese Knochenschrauben 10, die vorzugsweise auch aus dem gleichen Kunststoffmaterial bestehen, weisen einen Gewindeschaft 11 und einen verbreiterten Kopf 12 auf. Der Kopf 12 weist bei dem in Figur 3 dargestellten Ausführungsbeispiel an seiner Unterseite radial nach außen schräg abfallende Spannflächen 13 auf, deren Neigung im dargestellten Ausführungsbeispiel der Neigung der ebenfalls schräg nach außen abfallenden Anlagefläche 4 entspricht. Der Außendurchmesser des Kopfes 12 ist kleiner als der Außendurchmesser der zylindrischen Wand 5, ebenso ist der Außendurchmesser der Anlagefläche 4 größer als der Durchmesser der Spannfläche 13. Der Durchmesser der Durchstecköffnung 2 ist größer als der Außendurchmesser des Gewindeschaftes 11, die Knochenschraube ist also mit Spiel in die Durchstecköffnung 2 eingesetzt.

Beim Spannen der Knochenschraube, das durch Einsetzen eines geeigneten, in der Zeichnung nicht dargestellten Werkzeugs in eine Einsetzöffnung 14 auf der Oberseite des Kopfes 12 erfolgt, legt sich die Spannfläche 13 an die Anlagefläche 4 der Knochenplatte an und drückt diese dadurch gegen die Oberfläche des Knochens 9. Dabei werden gleichzeitig radial nach innen gerichtete Kräfte auf die Knochenplatte 1 übertragen, die zu einer Erhöhung der Festigkeit der Knochenplatte in diesem Bereich beitragen. Die Durchstecköffnung 2 wird durch den Kopf gleichsam überbrückt.

Wie aus Figur 3 ersichtlich ist, muß die Knochenschraube nicht genau senkrecht zur Knochenplatte eingeschraubt werden, sondern es sind auch geringe Neigungen gegenüber der Senkrechten möglich. Dabei überdecken sich die Anlagefläche und die Spannfläche auf einer Seite der Knochenschraube mehr als auf der gegenüberliegenden Seite, trotzdem ergibt sich eine allseitige Anlage zwischen Anlagefläche und Spannfläche und damit die gewünschte Beaufschlagung des Randes der Durchstecköffnung 2 mit nach innen gerichteten Kräften.

In den in Figur 3 dargestellten Ausführungsbeispielen ist die Spannfläche 13 nicht Teil des Kopfes 12, sondern die Spannfläche 13 ist Teil eines Ringes 15, der an der Unterseite des Kopfes 12 angeordnet und mit diesem dauerhaft verbunden ist, beispielsweise durch Verklebung oder Verschweißung.

Selbstverständlich wäre es auch möglich, die Spannfläche 13 unmittelbar an die Unterseite des Kopfes 12 anzuformen.

## Patentansprüche

1. Osteosyntheseimplantat mit einer Knochenplatte mit Durchstecköffnungen und mit schrauben-, zuganker- oder stiftförmigen Spannelementen, die in die Durchstecköffnungen eingesetzt sind und mit einem verbreiterten Kopf am Rand der Durchstecköffnungen anliegen und dadurch die Knochenplatte gegen einen Knochen spannen,
dadurch gekennzeichnet, daß die Ränder der Durchstecköffnungen (2) eine radial nach außen abfallende Anlagefläche (4) bilden, daß die verbreiterten Köpfe (12) der Spannelemente (10) über eine radial nach außen abfallende Spannfläche (13) an der Anlagefläche anliegen, daß der Außenumfang der Anlagefläche (4) größer ist als der Außenumfang der Spannfläche (13) und daß das Spannelement (10) die Durchstecköffnung (2) mit so viel Spiel durchsetzt, daß es gegenüber der Senkrechten auf der Knochenplatte (1) mit einer Neigung, bei der sich trotzdem eine allseitige Anlage zwischen Anlagefläche und Spannfläche ergibt, in die Durchstecköffnung (2) einsetzbar ist.

2. Implantat nach Anspruch 1, dadurch gekennzeichnet, daß die Spannfläche (13) Teil des Kopfes (12) des Spannelements (10) ist.

3. Implantat nach Anspruch 1, dadurch gekennzeichnet, daß die Spannfläche (13) Teil eines Ringes (15) ist, der zwischen Anlagefläche (4) und Kopf (12) des Spannelements (11) eingelegt ist.

4. Implantat nach Anspruch 3, dadurch gekennzeichnet, daß der Ring (15) mit der Unterseite des Kopfes (12) des Spannelements (10) dauerhaft verbunden ist.

5. Implantat nach einem der Ansprüche 1 bis 4, dadurch gekennzeichnet, daß die Neigungen der Anlagefläche (4) und der Spannfläche (13) gleich sind.

6. Implantat nach einem der voranstehenden Ansprüche, dadurch gekennzeichnet, daß die Anlagefläche (4) gegenüber der Außenfläche (3) der Knochenplatte (1) in axialer Richtung des Spannelements (10) zurückgesetzt ist.

7. Implantat nach einem der voranstehenden Ansprüche, dadurch gekennzeichnet, daß die Knochenplatte (1) auf ihrer Unterseite Bereiche geringerer Plattenstärke aufweist, die zwischen benachbarten Durchstecköffnungen (2) angeordnet sind, sich jedoch nicht bis in den Bereich des Randes der Durchstecköffnungen (2) und nicht bis in den Bereich des Längsrandes (7) der Knochenplatte (1) erstrecken.

8. Implantat nach einem der voranstehenden Ansprüche, dadurch gekennzeichnet, daß Knochenplatte (1) und Spannelement (10) aus Kunststoff bestehen.

9. Implantat nach Anspruch 8, dadurch gekennzeichnet, daß der Kunststoff resorbierbar ist.

## Claims

1. An implant for osteosynthesis, comprising a bone plate having passage openings and comprising screw, bolt or pin-type clamping members which are inserted into the passage openings and rest against the edge thereof by means of a broadened head and thereby clamp the bone plate to a bone, characterised in that the edges of the passage openings (2) form a contact surface (4) sloping downwards and radially outwards, in that the broadened heads (12) of the clamping members (10) rest against the contact surface by means of a clamping surface (13) sloping downwards and radially outwards, in that the outer circumference of the contact surface (4) is greater than the outer circumference of the clamping surface (13) and in that the clamping member (10) extends through the passage opening (2) with sufficient play to enable it to be inserted into the passage opening (2) at an angle to the normal to the bone plate (1) at which all-round contact is still produced between the contact surface and the clamping surface.

2. An implant according to claim 1, characterised in that the clamping surface (13) is part of the head (12) of the clamping member (10).

3. An implant according to claim 1, characterised in that the clamping surface (13) is part of a ring (15) inserted between the contact surface (4) and the head (12) of the clamping member (11).

4. An implant according to claim 3, characterised in that the ring (15) is permanently connected to the underside of the head (12) of the clamping member (10).

5. An implant according to any one of claims 1 to 4, characterised in that the angles of the contact surface (4) and the clamping surface (13) are equal.

6. An implant according to any one of the preceding claims, characterised in that the contact surface (4) is set back in the axial direction of the clamping member (10) relative to the outer surface (3) of the bone plate (1).

7. An implant according to any one of the preceding claims, characterised in that the underside of the bone plate (1) has regions of reduced thickness arranged between adjacent passage openings (2), but not extending as far as the region of the edge of the passage openings (2), nor as far as the region of the longitudinal edge (7) of the bone plate (1).

8. An implant according to any one of the preceding claims, characterised in that the bone plate (1) and the clamping member (10) comprise plastics.

9. An implant according to claim 8, characterised in that the plastics is resorbable.

## Revendications

1. Implant d'ostéosynthèse comportant une plaque à os avec des ouvertures traversantes et des éléments de serrage en forme de vis, de tirants ou de broches, qui sont enfichés dans les ouvertures traversantes et reposent avec une tête élargie sur le bord des ouvertures traversantes de manière à serrer la plaque à os contre un os, caractérisé en ce que les bords des ouvertures traversantes (2) forment une surface d'appui (4) inclinée radialement vers l'extérieur, en ce que les têtes élargies (12) des éléments de serrage (10) sont en appui sur la surface d'appui par l'intermédiaire d'une surface de serrage (13) inclinée radialement vers l'extérieur, en ce que la périphérie extérieure de la surface d'appui (4) est supérieure à la périphérie extérieure de la surface de serrage (13) et en ce que l'élément de serrage (10) traverse l'ouverture traversante (2) avec un tel jeu qu'il est susceptible d'être mis en place dans l'ouverture traversante (2) par rapport à la verticale sur la plaque à os (1), avec une inclinaison qui permet malgré tout d'obtenir un appui de tous côtés entre la surface d'appui et la surface de serrage.

2. Implant selon la revendication 1, caractérisé en ce que la surface de serrage (13) fait partie de la tête (12) de l'élément de serrage (10).

3. Implant selon la revendication 1, caractérisé en ce que la surface de serrage (13) fait partie d'une bague (15) qui est insérée entre la surface d'appui et la tête (12) de l'élément de serrage (11).

4. Implant selon la revendication 3, caractérisé en ce que la bague (15) est reliée de manière durable à la face inférieure de la tête (12) de l'élément de serrage (10).

5. Implant selon l'une quelconque des revendications 1 à 4, caractérisé en ce que les inclinaisons de la surface d'appui (4) et de la surface de serrage (13) sont égales.

6. Implant selon l'une quelconque des revendications précédentes, caractérisé en ce que la surface d'appui (4) est en retrait par rapport à la surface extérieure (3) de la plaque à os (1) en direction axiale de l'élément de serrage (10).

7. Implant selon l'une quelconque des revendications précédentes, caractérisé en ce que la plaque à os (1) présente sur sa face inférieure des régions d'épaisseur de plaque plus minces qui sont agencées entre des ouvertures traversantes (2) voisines, mais qui ne s'étendent pas jusque dans la région du bord des ouvertures traversantes (2), ni jusque dans la région du bord longitudinal (7) de la plaque à os (1).

8. Implant selon l'une quelconque des revendications précédentes, caractérisé en ce que la plaque à os (1) et l'élément de serrage (10) sont en matière plastique.

9. Implant selon la revendication 8, caractérisé en ce que la matière plastique est résorbable.
